# EUROPEAN PATENT APPLICATION

(11) **EP 4 331 567 A1**
(43) Date of publication of application: **06.03.2024**
(21) Application number: 22796059.8
(22) Date of filing: 25.04.2022
(51) Int. Cl.: A61K 8/9722, A61K 8/64, A61Q 19/02, A61Q 19/08

(54) **COSMETIC COMPOSITION COMPRISING CHLORELLA PROTOTHECOIDES EXTRACT FOR SKIN WHITENING OR WRINKLE IMPROVEMENT AND PREPARATION METHOD THEREFOR**

(30) Priority: 26.04.2021 KR 20210053917
(71) Applicant: Daebong LS Co., Ltd., Incheon 21697 (KR); Daesang Corporation, Seoul 03130 (KR)
(72) Inventor: KIM, Wang-Bae, Yongin-si, Gyeonggi-do 16951 (KR); IM, Dong-Joong, Seoul 06704 (KR); KIM, Keon-Woo, Incheon 21910 (KR); PARK, Jin-Oh, Seoul 06276 (KR); KIM, Bo-Ra, Yongin-si, Gyeonggi-do 17003 (KR); HWANG, Ji-Eun, Goyang-si, Gyeonggi-do 10360 (KR)
(74) Representative: Plasseraud IP
(86) International application number: PCT/KR2022/005839
(87) International publication number: WO 2022/231226

(57) **Abstract**

The present disclosure relates to a cosmetic composition for skin whitening or wrinkle improvement, which contains a *Chlorella protothecoides* extract, and a method for preparing the same. The composition containing the *Chlorella protothecoides* extract of the present disclosure may be widely used as a cosmetic for skin whitening or wrinkle improvement since the problem of limited application as a cosmetic composition due to the characteristic fishy smell and dark color of the composition using *Chlorella* has been resolved.

## Description

### [Technical Field]

The present disclosure relates to a cosmetic composition for skin whitening or wrinkle improvement, which contains a *Chlorella protothecoides* extract, and a method for preparing the same. This application claims the priority of Korean Patent Application No. 10-2021-0053917, filed on April 26, 2021, the contents of which in their entirety are herein incorporated by reference.

### [Background Art]

Microalgae are photosynthetic organisms that synthesize organic materials, which are the major energy factors, and produce oxygen using carbon dioxide, water and solar energy. Since the microalgae can fix carbon dioxide in the atmosphere and have high protein and lipid contents, they can be used as food, feed or biomass for fuel production. Specifically, the primary metabolites of microalgae, such as lipids, glucides, proteins, etc., can be used as sources of various biofuels such as biodiesel, bioethanol or biogas and as raw materials of polyunsaturated fatty acids (PUFAs). And, the secondary metabolites of microalgae, which are high-value useful materials such as vitamins, carotenoids, polysaccharides, lutein, etc., can be used as functional foods, natural pigments, medicinal substances, animal feeds, aquaculture feeds, etc.

Conventionally, the use of *Chlorella* as a raw material of a cosmetic composition or a composition for external use has been limited due to its characteristic fishy smell and the dark green color derived from chlorophylls. However, *Chlorella protothecoides* can be used in a cosmetic composition since it has pale yellow color and light odor. Korean Patent Publication No. 10-2017-0023065 discloses a method of extracting soluble proteins from *Chlorella protothecoides.*

However, research and development of a *Chlorella* extract with the physiological activities of skin whitening, wrinkle improvement and antiaging that can be used as a cosmetic composition is insufficient.

### [References of Related Art]

### [Patent Documents]

(Paten document 0001) Korean Patent Publication No. 10-2017-0023065 (2017.03.02).

### [Disclosure]

### [Technical Problem]

The inventors of the present disclosure have researched to solve the problem that the use of *Chlorella* as a cosmetic composition is limited due to its characteristic fishy smell and dark color. As a result, they have found out that the problem can be solved and superior skin-whitening or wrinkle-improving effect can be achieved when *Chlorella protothecoides* is used and, thus, have completed the present disclosure.

Accordingly, the present disclosure is directed to providing a cosmetic composition for skin whitening or wrinkle improvement, which contains a *Chlorella protothecoides* extract, and a method for preparing the same.

### [Technical Solution]

The present disclosure provides a cosmetic composition for skin whitening or wrinkle improvement, which contains a *Chlorella protothecoides* extract.

The *Chlorella protothecoides* extract may contain a low-molecular-weight peptide.

The low-molecular-weight peptide may be obtained using a protease.

The protease may be derived from papaya.

The present disclosure also provides a method for preparing a *Chlorella protothecoides* extract, which includes:
a step of obtaining a dispersion by dispersing *Chlorella protothecoides* in a solvent;
a step of obtaining a reaction solution by adding a protease to the dispersion; and
a step of obtaining an extract containing a low-molecular-weight peptide from the reaction solution.

The dispersion may be achieved by compressing at 100-5000 psi.

10-150 parts by weight of the protease may be added based on 100 parts by weight of the *Chlorella protothecoides.*

The method may further include a step of obtaining a fraction by fractionating the extract with an ultrafiltration membrane.

### [Advantageous Effects]

A composition containing a *Chlorella protothecoides* extract of the present disclosure may be widely used as a cosmetic for skin whitening or wrinkle improvement since the problem of limited application as a cosmetic composition due to the characteristic fishy smell and dark color of the composition using *Chlorella* has been resolved.

### [Brief Description of Drawings]

FIG. 1 shows a result of testing tyrosinase-inhibiting effect by treating with samples prepared in Example 1 under different reaction conditions.
FIG. 2 shows a result of testing radical-scavenging effect by treating with a sample having the most superior tyrosinase-inhibiting effect in Test Example 1.
FIG. 3 shows a result of comparing the tyrosinase-inhibiting effect of extracts prepared by treating with papain and Flavourzyme.
FIG. 4 shows a result of comparing the radical-scavenging effect of extracts prepared by treating with papain and Flavourzyme.
FIG. 5 shows a result of investigating intracellular melanin production-inhibiting effect by treating with a sample having the most superior tyrosinase-inhibiting effect in Test Example 1 at different concentrations.
FIG. 6 shows a result of investigating intracellular collagen synthesis by treating with a sample having the most superior tyrosinase-inhibiting effect in Test Example 1 at different concentrations.
FIG. 7 shows a result of investigating intracellular collagen synthesis by treating with a sample having the most superior tyrosinase-inhibiting effect in Test Example 1 at different concentrations.
FIG. 8 shows a result of investigating cellular proliferation by treating with a sample having the most superior tyrosinase-inhibiting effect in Test Example 1 at different concentrations.

### [Best Mode]

Hereinafter, the present disclosure will be described in detail. Prior to the description, it should be understood that the terms or words used in the present specification and claims should not be construed as being limited to ordinary or dictionary meanings and should be interpreted as meanings and concepts consistent with the technical idea of the present disclosure based on the principle that an inventor can appropriately define the concept of terms in order to explain his/her invention in the best way. Therefore, it should be understood that, since the exemplary embodiments described in the present specification are merely specific exemplary embodiments of the present disclosure and do not represent all of the technical ideas of the present disclosure, various equivalents and modifications can replace them at the time of this application.

The present disclosure is characterized by a cosmetic composition for skin whitening or wrinkle improvement, which contains a *Chlorella protothecoides* extract.

*Chlorella* is a genus of microalgae corresponding to single-celled green algae. They are important green algae that proliferate rapidly by assimilating carbon sources (carbon dioxide in the air), nitrogen and other minerals through photosynthesis, just like the physiological function of general plants. *Chlorella* is important as an organic source because it contains a lot of proteins and useful amino acids as well as fats and other active ingredients. Therefore, it is attracting attentions as a substance that can be a protein ad fat source.

The microalgae in the genus *Chlorella* used in the present disclosure may be *Chlorella protothecoides.*

The *Chlorella protothecoides* extract may contain a low-molecular-weight peptide.

The low-molecular-weight peptide may have a molecular weight of 0.1-10 kDa, specifically 0.1-5 kDa, more specifically 0.1-1 kDa. If the molecular weight is lower than 0.1 kDa, a lot of cost and time are required for low-molecularization. And, if it is higher than 1 kDa, the efficiency of transdermal penetration is decreased.

The low-molecular-weight peptide may be obtained using a protease.

As the protease, one or more commercially available enzyme may be used. The commercially available enzyme may be one or more selected from Kojizyme, Alcalase, Esperase, Neutrase, Flavourzyme, etc. Specifically, an enzyme derived from papaya may be used.

The papaya-derived enzyme may be any plant protease derived from papaya. Specifically, papain may be used.

The papain is a protein hydrolase obtained from the latex of the fruit of papaya tree, which is native to South America. The papain enzyme has a molecular weight (Mw) of 20,000-30,000, an isoelectric point of pH 8.75 and an optimal reaction pH of 6-7. It is stable at pH 4.0-8.0 and can produce peptone by degrading proteins (by acting as an endopeptidase).

The papain can be prepared by purifying papain latex which has been obtained by making shallow cuts on the skin of immature fruit or trunk of papaya, collecting the latex dropping from the cuts and leaving it for 4-6 hours to solidify.

The papain is used effectively in the food industry for softening of meat, removal of precipitated particles generated during beer refrigeration, etc. and is also used as a medicine. In addition, the papain has an effect of softening meat since it has strong hydrolytic power against connective tissue collagen, elastin, actomyosin, etc.

The *Chlorella protothecoides* extract may be contained in the cosmetic composition at a concentration of 50-1000 µg/mL, specifically 200-400 µg/mL.

If the concentration of the *Chlorella protothecoides* extract in the cosmetic composition is below 50 µg/mL, the effect of the *Chlorella protothecoides* extract of moisturizing skin, improving skin elasticity, regenerating skin and alleviating skin irritation may not be achieved appropriately. And, if the concentration of the *Chlorella protothecoides* extract in the cosmetic composition exceeds 1000 µg/mL, the effect of other ingredients in the cosmetic composition may be negatively affected without further increase in the effect of the *Chlorella protothecoides* extract.

The cosmetic composition of the present disclosure may contain ingredients commonly used in cosmetic compositions, for example, common adjuvants such as an antioxidant, a stabilizer, a solubilizer, a vitamin, a pigment and a flavorant and a carrier.

The cosmetic composition of the present disclosure may be prepared into any formulation commonly used in the art. For example, it may be formulated into a solution, a suspension, an emulsion, a paste, a gel, a cream, a lotion, a powder, a soap, a surfactant-containing cleanser, an oil, a powder foundation, an emulsion foundation, a wax foundation, a spray, etc., although not being limited thereto.

More specifically, it may be formulated into a softening lotion, a nourishing lotion, a nourishing cream, a massage cream, an essence, an eye cream, a cleansing cream, a cleansing foam, a cleansing water, a pack, a spray or a powder.

When the formulation of the present disclosure is a paste, a cream or a gel, an animal oil, a plant oil, a wax, paraffin, starch, tragacanth, a cellulose derivative, polyethylene glycol, silicone, bentonite, silica, talc, zinc oxide, etc. may be used as a carrier ingredient.

When the formulation of the present disclosure is a powder or a spray, lactose, talc, silica, aluminum hydroxide, calcium silicate or polyamide powder may be used as a carrier ingredient. Especially, a spray may further contain a propellant such as chlorofluorohydrocarbon, propane/butane or dimethyl ether.

When the formulation of the present disclosure is a solution or an emulsion, a solvent, a solubilizer or an emulsifier may be used as a carrier ingredient. For example, water, ethanol, isopropanol, ethyl carbonate, ethyl acetate, benzyl alcohol, benzyl benzoate, propylene glycol, 1,3-butylene glycol, glycerol aliphatic ester, polyethylene glycol or sorbitan fatty acid ester may be used.

When the formulation of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier ingredient.

When the formulation of the present disclosure is a surfactant-containing cleanser, an aliphatic alcohol sulfate, an aliphatic alcohol ether sulfate, sulfosuccinic monoester, isethionate, an imidazolinium derivative, methyl taurate, sarcosinate, a fatty acid amide ether sulfate, an alkylamidobetaine, an aliphatic alcohol, a fatty acid glyceride, a fatty acid diethanolamide, vegetable oil, a lanolin derivative, an ethoxylated glycerol fatty acid ester, etc. may be used as a carrier ingredient.

When the formulation of the present disclosure is a soap, a surfactant-containing cleanser or a non-surfactant-containing cleanser, it may be applied to skin and then wiped, detached or washed with water. Specifically, the soap may be a liquid soap, a powder soap, a solid soap or an oil soap, the surfactant-containing cleanser may be a cleansing foam, a cleansing water, a cleansing towel or a cleansing pack, and the non-surfactant-containing cleanser may be a cleansing cream, a cleansing lotion, a cleansing water or a cleansing gel, although not being limited thereto.

The present disclosure is also characterized by a method for preparing a *Chlorella protothecoides* extract, which includes: a step of obtaining a dispersion by dispersing *Chlorella protothecoides* in a solvent; a step of obtaining a reaction solution by adding a protease to the dispersion; and a step of obtaining an extract containing a low-molecular-weight peptide from the reaction solution.

When the *Chlorella protothecoides* is dispersed in the solvent, the dispersion may be accomplished by compressing the mixture of the *Chlorella protothecoides* and the solvent.

The compression may allow more effective extraction of active ingredients contained in the *Chlorella protothecoides* by inducing the damage or stimulation of the cell wall of the *Chlorella protothecoides.*

The compression may be performed at a pressure of 100-5000 psi, specifically 500-3000 psi, more specifically 1000-2000 psi.

If the pressure of the compression is below 100 psi, the extraction efficiency of the active ingredients may not be improved significantly due to decreased compression efficiency. And, if it exceeds 5000 psi, it may be difficult to control temperature and the active ingredients may be destroyed.

The dispersion may be obtained by adding of 10-30 times of the solvent based on the amount of the *Chlorella protothecoides.*

As the solvent, purified water, a lower alcohol such as ethanol, methanol and butanol or a mixture solvent thereof may be used. Specifically, purified water may be used.

Next, a reaction solution may be obtained by adding a protease to the dispersion. In addition, another solvent may be further added to the dispersion in addition to the protease.

The addition amount of the protease may be 1-100 parts by weight, specifically 20-70 parts by weight, based on 100 parts by weight of the *Chlorella protothecoides.* If the content of the protease is less than 1 part by weight based on 100 parts by weight of the *Chlorella protothecoides,* the reaction time may be increased due to low physiological activity. And, if it exceeds 100 parts by weight, cost may be increased due to low efficiency.

The additional solvent may be purified water, a lower alcohol such as ethanol, methanol and butanol or a mixture solvent thereof. Specifically, purified water may be used and it may be added in an amount of 10-100 times based on the *Chlorella protothecoides.*

If the addition amount of the additional solvent is less than 10 times of the *Chlorella protothecoides,* it is difficult to selectively separate unreacted *Chlorella protothecoides* and a low-molecular-weight peptide because it is difficult to effectively precipitate the unreacted *Chlorella protothecoides.* And, if it exceeds 100 times, cost is excessive because a large reaction vessel is required and the amount of the reaction solution is too large.

The reaction solution may be stirred at 40-60 °C for 6-30 hours, specifically at 45-55 °C for 24-28 hours.

Then, an extract containing a low-molecular-weight peptide may be obtained from the reaction solution by centrifuging the reaction solution and then filtering a supernatant.

The filtration may be performed using a filtration membrane having a pore size of 0.1-0.3 µm, specifically 0.15-0.25 µm.

In addition, the present disclosure may further include a step of obtaining a fraction by fractionating the extract with an ultrafiltration membrane.

The ultrafiltration membrane may be one prepared from a polymer selected from a group consisting of cellulose acetate, a polyacrylonitrile-co-polyvinyl chloride copolymer (PAN-co-PVC), polysulfone, polyethersulfone (PES), polyvinylidene fluoride (PVDF), an aliphatic polyamide, an aromatic polyamide, polyimide, polyetherimide and polyether ether ketone.

In addition, the ultrafiltration membrane used for the ultrafiltration may have a molecular weight cut-off of 10 kDa or lower, 5 kDa or lower, 3 kDa or lower, 1 kDa or lower or 0.6 kDa or lower, specifically 1 kDa or lower.

Hereinafter, the present disclosure will be described in more detail through examples and test examples. However, the examples of the present disclosure may be changed into various different forms and the scope of the present disclosure is not limited to the examples. The examples of the present disclosure are provided so that the present disclosure is more completely described to those having ordinary knowledge in the art.

### Example 1: Preparation of extract containing low-molecular-weight peptides of Chlorella protothecoides

A dispersion was prepared by mixing 5 w/w% of *Chlorella protothecoides* with purified water and compressing at 1500 psi. A reaction solution was prepared by adding 50 parts by weight of papain and 6000 parts by weight of purified water based on 100 parts by weight of the *Chlorella protothecoides* and stirring the mixture. After heating the reaction solution at 40 °C, 50 °C or 60 °C for 6 hours, 12 hours, 24 hours or 28 hours, the solution was filtered through a filtration membrane with a pore size of 0.2 µm. Then, an extract containing low-molecular-weight peptides of 1 kDa or lower was prepared by fractionating with a 1-kDa ultrafiltration membrane.

### Test Example 1: Tyrosinase inhibition effect of extract

In order to investigate the skin-whitening effect of the extract containing low-molecular-weight peptides of *Chlorella protothecoides* prepared in Example 1, the degree of inhibition of the function of tyrosinase was investigated.

Tyrosinase is an enzyme which helps the production of melanin by promoting the oxidation of tyrosine in the body. In this test example, the skin-whitening effect was evaluated by the method of measuring the formation of melanin by oxidation of tyrosine as a result of inhibition of the enzyme (SH Pomerantz, Biol Chem, 241: 161-168, 1966).

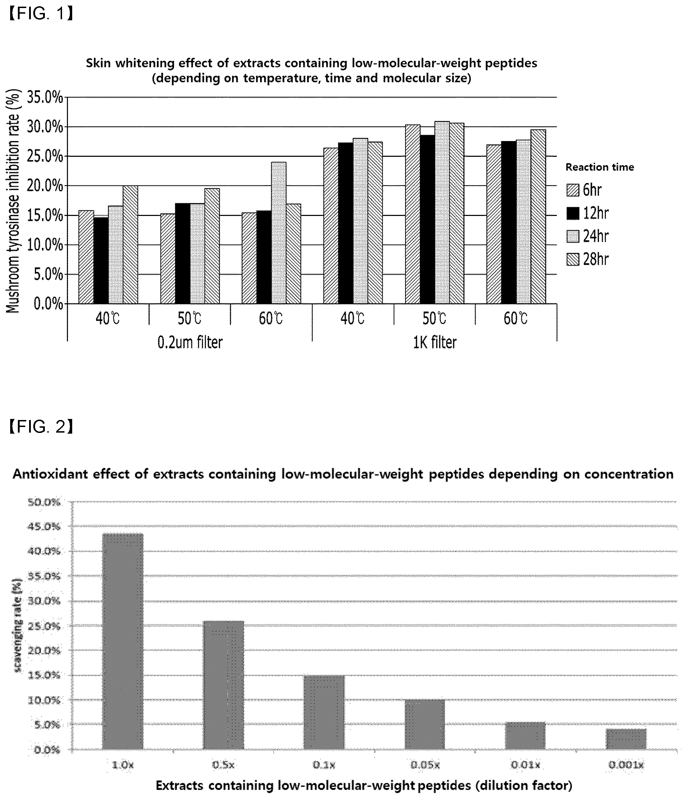

Since the concentration dependence of the inhibitory effect was not confirmed at high concentrations, the extract containing low-molecular-weight peptides of *Chlorella protothecoides* was diluted 5 times for evaluation of tyrosinase inhibition. After placing L-tyrosine in a test tube as an enzyme reaction substrate, the extract prepared in Example 1 was added and reaction was conducted at 37 °C for 15 minutes. Then, after adding mushroom tyrosinase and conducting reaction at 37 °C for 15 minutes, absorbance was measured at 475 nm. The result is shown in FIG. 1.

As shown in FIG. 1, the low-molecular-weight peptide extract obtained by reaction at 50 °C for 24 hours and fractionation with a 1-kDa ultrafiltration membrane showed the most superior effect of inhibiting tyrosinase activity. The following experiments were performed for the low-molecular-weight peptide extract to confirm additional effects.

### Test Example 2: Measurement of antioxidant effect of extract by DPPH method

In order to investigate the antioxidant effect of the extract containing low-molecular-weight peptides of *Chlorella protothecoides* which showed the most superior tyrosinase activity-inhibiting effect in Test Example 1, free radical-scavenging rate was measured by the DPPH method.

The DPPH method measures antioxidant activity based on the power of reducing the free radical called DPPH (2,2-di(4-tert-octylphenyl)-1-picrylhydrazyl). It measures free radical-scavenging rate at a wavelength of 560 nm by comparing the decrease of absorbance due to reduction of DPPH by a test substance with that of a blank test solution.

Specifically, after preparing a 60 µM standard DPPH solution, samples were prepared by diluting the extract that showed the highest tyrosinase-inhibiting effect in Test Example 1 to 100-0.1%. Then, after mixing the samples with the standard DPPH solution at the same ratio and stirring the mixture, reaction was conducted at 37 °C for 30 minutes and absorbance was measured at 560 nm. The result is shown in FIG. 2.

As shown in FIG. 2, the extract containing low-molecular-weight peptides of *Chlorella protothecoides* showed antioxidant effect in a concentration-dependent manner.

### Test Example 3: Comparison of effect depending on protease

For comparison of effect depending on protease, the degree of inhibition of tyrosinase activity and free radical scavenging rate of the extract containing low-molecular-weight peptides of *Chlorella protothecoides* were measured by treating with different proteases.

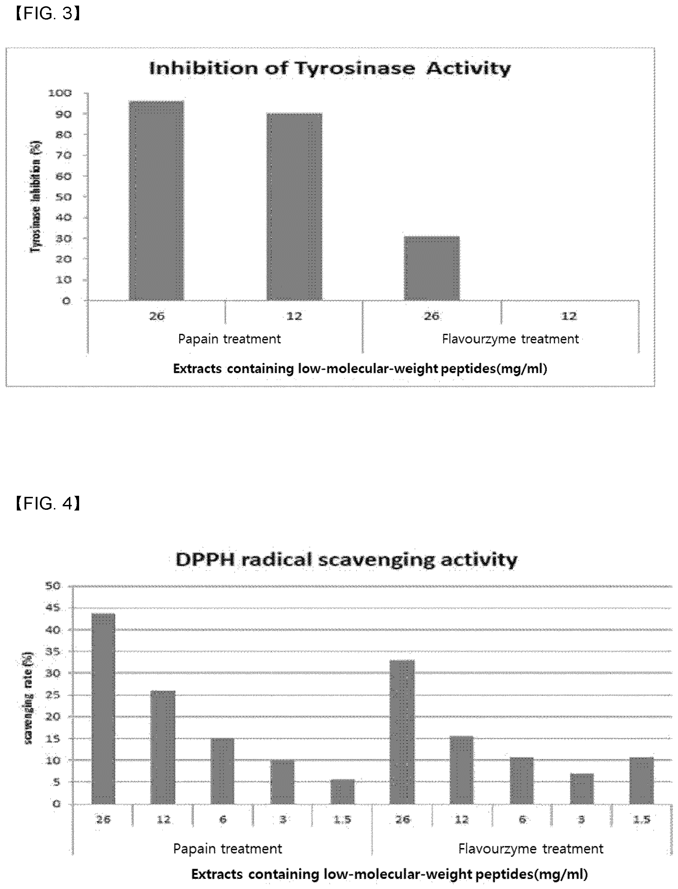

A dispersion was prepared by mixing 5 w/w% of *Chlorella protothecoides* with purified water and compressing at 1500 psi. Two reaction solutions were prepared by adding 6000 parts by weigh of purified water based on 100 parts by weight of the *Chlorella protothecoides* to the dispersion and treating with 50 parts by weight of papain or Flavourzyme and stirring the mixture. After heating the two reaction solutions at 50 °C for 24 hours, the solutions were filtered through a filtration membrane with a pore size of 0.2 µm. Then, extracts containing low-molecular-weight peptides of 1 kDa or lower were prepared by fractionating with a 1-kDa ultrafiltration membrane. After placing L-tyrosine in test tubes as an enzyme reaction substrate, the extracts were added and reaction was conducted at 37 °C for 15 minutes. Then, after adding mushroom tyrosinase and conducting reaction at 37 °C for 15 minutes, absorbance was measured at 475 nm. The result is shown in FIG. 3. After preparing a 60 µM standard DPPH solution, samples were prepared by mixing the samples with the standard DPPH solution at the same ratio and stirring the mixture. Then, reaction was conducted at 37 °C for 30 minutes and absorbance was measured at 560 nm. The result is shown in FIG. 4.

As shown in FIG. 3, the extract containing low-molecular-weight peptides extracted by treating with papain showed about 3.3 times higher tyrosinase activity-inhibiting effect than that treated with Flavourzyme. Also, as shown in FIG. 4, the extract treated with papain showed about 1.35-1.68 times superior antioxidant effect.

### Test Example 4: Measurement of melanin production-inhibiting effect using B16F10 cells

In order to investigate the intracellular skin-whitening effect of the extract containing low-molecular-weight peptides of *Chlorella protothecoides* that showed the most superior tyrosinase activity-inhibiting effect in Test Example 1, the degree of inhibition of melanin production was measured for B16F10 cells.

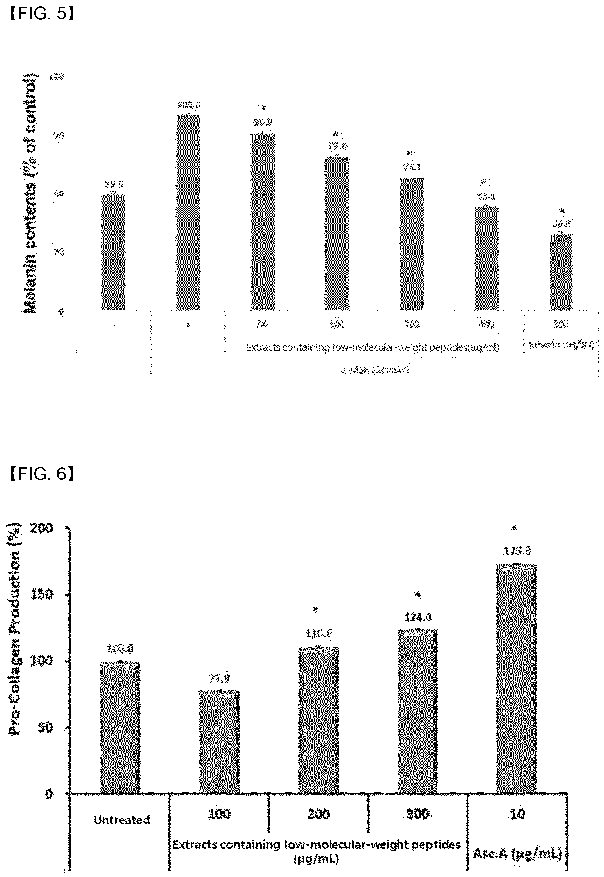

After dispensing B16F10 cells at 2.0×10⁴ cells/mL and culturing for 24 hours and then treating with α-MSH, which is a melanogenesis-inducing hormone, arbutin as a positive control or the extract at different concentrations was added. Then, after culturing the cells in a medium for 72 hours and extracting melanin by treating with 1 N NaOH at 80°C for 1 hour, absorbance was measured at 405 nm. The result is shown in FIG. 5.

As shown in FIG. 5, the extract containing low-molecular-weight peptides of *Chlorella protothecoides* showed superior effect of inhibiting melanin synthesis in a concentration-dependent manner.

### Test Example 5: Wrinkle-improving and antiaging effects of extract through collagen production

In order to investigate the wrinkle-improving and antiaging effects of the extract containing low-molecular-weight peptides of *Chlorella protothecoides* that showed the most superior tyrosinase activity-inhibiting effect in Test Example 1, intracellular production of collagen was investigated.

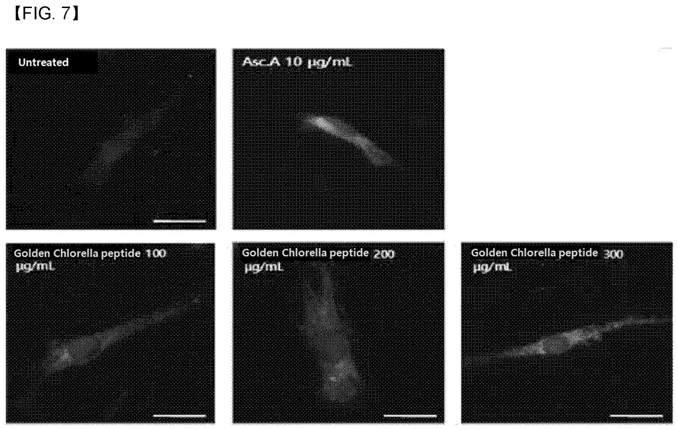

In order to investigate the effect on collagen production, human dermal fibroblasts (NHDFs) were dispensed at 2.0×10⁴ cells/mL and cultured for 24 hours. After culturing the cells in a medium to which ascorbic acid (Asc.A) as a positive control or the extract at different concentrations was added for 48 hours, the amount of procollagen type I C-peptide was measured. In addition, the cells were stained with 1 µg/mL DAPI, which is a fluorescence stain, for 30 minutes in the dark and observed under an optical microscope. The result is shown in FIGS. 6 and 7.

As shown in FIGS. 6 and 7, the collagen production was increased and the fluorescence intensity became stronger as the concentration of the extract was increased, indicating that the extract containing low-molecular-weight peptides of *Chlorella protothecoides* has superior wrinkle-improving and antiaging effects.

### Test Example 6: Cellular differentiation-promoting effect of extract

Wound healing assay was carried out to investigate the cellular differentiation-promoting effect of the extract containing low-molecular-weight peptides of *Chlorella protothecoides* that showed the most superior tyrosinase activity-inhibiting effect in Test Example 1.

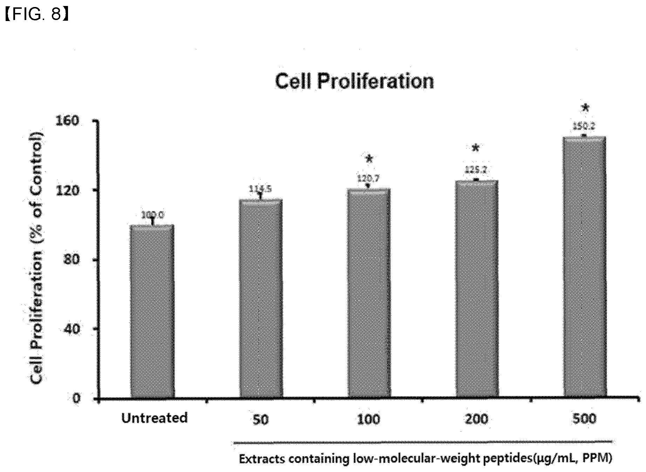

Wound healing assay is a test method that investigates the wound healing activity of a sample by creating a wound in a single layer of cells and monitoring the decrease in the gap over time using a time-lapse microscope. Human keratinocytes (HaCaT cells) were dispensed at 2.0×10⁵ cells/mL and cultured for 24 hours. After applying physical damage using a pipette tip, cell migration was monitored over time. The result is shown in FIG. 8.

As shown in FIG. 8, the extract showed superior cellular differentiation-promoting effect with the rate of cellular proliferation increased with concentration.

## Claims

1. A cosmetic composition for skin whitening or wrinkle improvement, comprising a *Chlorella protothecoides* extract.

2. The cosmetic composition for skin whitening or wrinkle improvement according to claim 1, wherein the *Chlorella protothecoides* extract comprises a low-molecular-weight peptide.

3. The cosmetic composition for skin whitening or wrinkle improvement according to claim 2, wherein the low-molecular-weight peptide is obtained using a protease.

4. The cosmetic composition for skin whitening or wrinkle improvement according to claim 3, wherein the protease is derived from papaya.

5. A method for preparing a *Chlorella protothecoides* extract, comprising:
a step of obtaining a dispersion by dispersing *Chlorella protothecoides* in a solvent;
a step of obtaining a reaction solution by adding a protease to the dispersion; and
a step of obtaining an extract comprising a low-molecular-weight peptide from the reaction solution.

6. The method for preparing a *Chlorella protothecoides* extract according to claim 5, wherein the dispersion is achieved by compressing at 100-5000 psi.

7. The method for preparing a *Chlorella protothecoides* extract according to claim 5, wherein 10-150 parts by weight of the protease is added based on 100 parts by weight of the *Chlorella protothecoides.*

8. The method for preparing a *Chlorella protothecoides* extract according to claim 5, which further comprises a step of obtaining a fraction by fractionating the extract with an ultrafiltration membrane.
